# EUROPEAN PATENT SPECIFICATION

(11) **EP 4 267 241 B1**
(45) Date of publication and mention of the grant of the patent: **05.03.2025**
(21) Application number: 21844813.2
(22) Date of filing: 21.12.2021
(51) Int. Cl.: A61F 2/04, A61M 29/02, A61N 5/06

(54) **DEVICE FOR THE TREATMENT OF ESOPHAGEAL STENOSIS**
VORRICHTUNG ZUR BEHANDLUNG VON SPEISERÖHRENSTENOSE
DISPOSITIF POUR LE TRAITEMENT DE LA STÉNOSE OESOPHAGIQUE

(30) Priority: 23.12.2020 IT 202000032342
(43) Date of publication of application: 01.11.2023
(73) Proprietor: Sidam S.r.l., 41037 S. Giacomo Roncole, Mirandola (MO) (IT)
(72) Inventor: AZZOLINI, Graziano, 41037 Mirandola (MO) (IT)
(74) Representative: Brunacci, Marco
(86) International application number: PCT/IB2021/062065
(87) International publication number: WO 2022/137100

(56) References cited:
- WO-A1-2015/186097
- WO-A1-2020/191400
- US-A- 5 445 608
- US-A1- 2004 073 088
- US-A1- 2012 071 867

## Description

### Technical Field

The present invention relates to a device for the treatment of esophageal stenosis.

### Background Art

As is well known, esophageal stenosis can be caused by a variety of factors such as, e.g., lesions caused by the ingestion of caustic substances, scarring lesions as a result of esophageal anastomosis due to atresia, post chemotherapy or radiotherapy or peptic lesions, etc.

In order to allow the recovery of the correct functionality of the organ, thus ensuring the patient a good quality of life, the tract of the esophagus affected by the stenosis is generally dilated.

These dilations must be repeated over time to address the inevitable problem of recurrence.

In order to avoid excessive dilatation, special devices, also called "removable stents", are used.

The devices of known type used to dilate the esophageal tract affected by the stenosis can be of two types: self-expanding type or to be introduced after dilation of the stenosis.

The devices of the self-expanding are adapted to compress the inner walls of the stenotic esophageal tract, thus preventing restenosis, and have the characteristic of allowing food to pass through them.

However, this type of device does not allow restoring, as a result of their removal, a sufficiently wide caliber of the esophagus and, above all, does not allow the same to recover the elasticity necessary to ensure the correct and autonomous functioning of the organ.

The second type of known device described above, on the other hand, is adapted to let food pass around the stent, i.e. between its outer wall and the inner wall of the esophagus.

In particular, the use of this type of device is aimed at forcing the esophagus to a continuous dilating activity so as to obtain a wider and, above all, more elastic caliber of the esophagus.

This type of known devices also has, however, its drawbacks.

In fact, food passing between the device and the wall of the esophagus may find it difficult to pass because of the reduced space available and its poor elasticity. This drawback, together with the sensation of retrosternal obstruction felt by the patient, can occur both in the periods immediately following the introduction of the device into the esophagus, as a result of the applied dilatation, and in the following period, due to the progressive recurrence of the stenosis and consequent reduction of the space useful for the passage of food and secretions.

The use of these known devices may therefore be stressful for the patient and for the esophagus itself, and aggravate an already delicate clinical situation, with consequent suffering for the patient.

From the patent document WO 2012/110891 is known, on the other hand, a special stent which has the characteristic of having a central tube, around which the food passes as in the case of the second type of devices mentioned above, and variable stiffness means associated externally to the central tube.

The variable stiffness means consist, in particular, of a flexible element, of tubular conformation, associated with the central tube so as to define an air chamber positioned between them and which can be filled with an operating fluid.

Even the stents shown in WO 2012/110891 and WO 2015/186097A1, however, are susceptible to refinements aimed, in particular, at simplifying and making the device cheaper while maintaining considerable convenience and efficiency of use.

Other devices are known from US 2004/073088 A1, US 2012/071867 A1, US 5 445 608 A and WO 2020/191400 A1.

### Description of the Invention

The main aim of the present invention is to devise a device for the treatment of esophageal stenosis which allows reducing the time required to reconstruct the esophageal wall by greatly decreasing the time the device stays inside the patient's esophagus.

Specifically, the present invention seeks to devise a device which allows food to pass between the device itself and the inner walls of the esophagus while causing no discomfort to the patient.

Additionally, the present invention is intended to minimize the risk of scar tissue activation and subsequent irritation or injury to the esophagus due to the presence of the device itself.

Within this aim, one object of the present invention is to devise a device which can adapt to the specific condition of the patient, so as to best optimize the applied therapy and, at the same time, not to cause any kind of additional discomfort for the patient, thus facilitating full recovery.

Still one object of the present invention is to devise a device which can be applied to the patient more easily than a device of known type.

Another object of the present invention is to devise a device for the treatment of esophageal stenosis which allows the mentioned drawbacks of the prior art to be overcome within a simple, rational, easy and effective to use as well as affordable solution.

The aforementioned objects are achieved by the present device for the treatment of esophageal stenosis, having the characteristics of claim 1.

### Brief Description of the Drawings

Other characteristics and advantages of the present invention will become more apparent from the description of some preferred, but not exclusive, embodiments of a device for the treatment of esophageal stenosis, illustrated by way of an indicative, yet non-limiting example, in the accompanying tables of drawings wherein:
Figure 1 is an exploded view of a first embodiment of the device according to the invention;
Figure 2 is an exploded view of a second embodiment of the device not covered by the wording of the claims,
Figure 3 is a longitudinal cross-sectional view of the device in Figure 2;
Figure 4 is an exploded view of a third embodiment of the device not covered by the wording of the claims,
Figure 5 is a longitudinal cross-sectional view of the device in Figure 4;

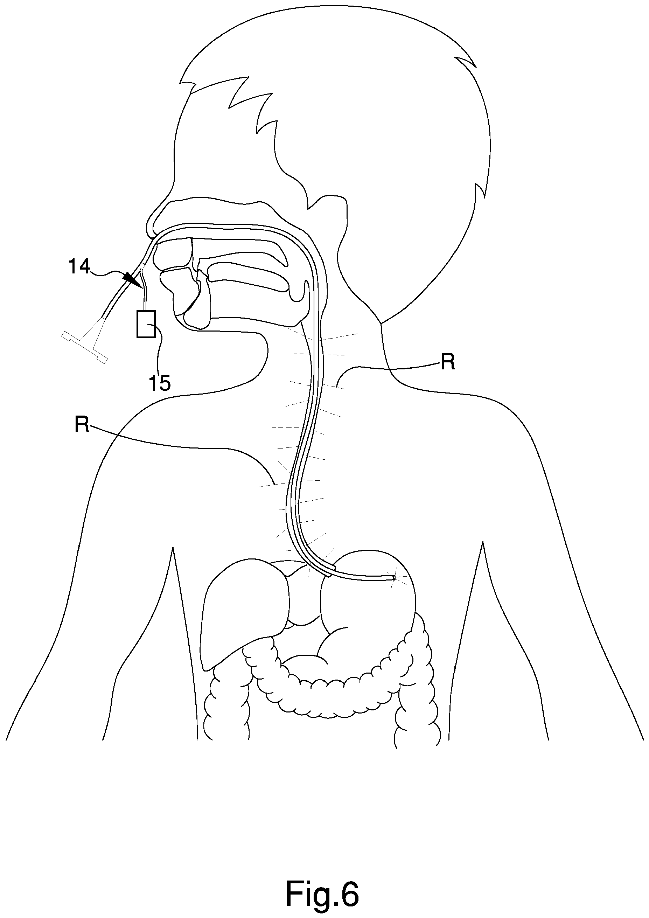

Figure 6 is a schematic representation of the device according to the invention during its use.

### Embodiments of the Invention

With particular reference to these figures, reference numeral 1 globally indicates a device for the treatment of esophageal stenosis.

Preferably, the aforementioned device is used for the treatment of benign esophageal stenosis.

The device 1 comprises at least one body 2 of elongated shape, intended to be inserted inside the esophagus of a patient at the stenotic tract to be treated.

The body 2, in particular, extends along a longitudinal direction L and is made of a material flexible enough to allow it to be introduced into the patient via the oral route.

In the embodiments shown in the figures, the body 2 has a substantially circular cross-section and is internally hollow, to define an inner conduit 3.

At least one tubular sheath 4 is mounted around the body 2 which has an inner diameter that is larger than the outer diameter of the body 2 so as to define an interspace 5 between the body 2 and the tubular sheath 4.

Preferably, the tubular sheath 4 also has a circular cross-section and is arranged substantially coaxial to the body 2; the interspace 5 therefore has a substantially annular conformation.

Advantageously, the tubular sheath 4 is transparent in color.

The device 1 comprises at least one connection element 6 positioned between the body 2 and the tubular sheath 4.

The connection element 6 is shaped so as to join the outer surface 4a of the tubular sheath 4 to the outer surface 2a of the body 2.

In more detail, the connection element 6 defines a joining surface 6a which is substantially sloped with respect to the outer surface 4a of the tubular sheath 4 and to the outer surface 2a of the body 2.

Such joining surface 6a is adapted to facilitate the passage of food in the space comprised between the device 1 and the inner wall of the esophagus.

Usefully, the device 1 comprises at least two connection elements 6 arranged at the axial ends of the tubular sheath 4.

Each of these two connection elements 6, thus arranged on opposite sides of the tubular sheath 4, has a respective substantially conical joining surface 6a.

More particularly, the joining surfaces 6a of the connection elements 6 converge outwardly and have opposite, i.e., mirror-like, inclinations to each other.

The connection elements 6 axially delimit the interspace 5 placed between the body 2 and the tubular sheath 4; it follows, therefore, that the interspace 5 is delimited internally by the body 2, externally by the tubular sheath 4 and axially by the connection elements 6.

In addition, the connection elements 6 respectively comprise an opening 10 adapted to allow the passage of the body 2.

The tight fixing between the connection elements 6, the body 2 and the tubular sheath 4 is ensured by the interposition of an adhesive substance 7, e.g. of the type of silicone paste and applied e.g. between the outer surface 2a of the body 2 and the inner surface of the connection elements 6, as well as between the inner surface of the tubular sheath 4 and the outer surface of the connection elements 6.

Furthermore, the device 1 comprises at least one stiffening element 8, 9 housed in the interspace 5 and comprising a plurality of ribs 8 extending along the interspace 5.

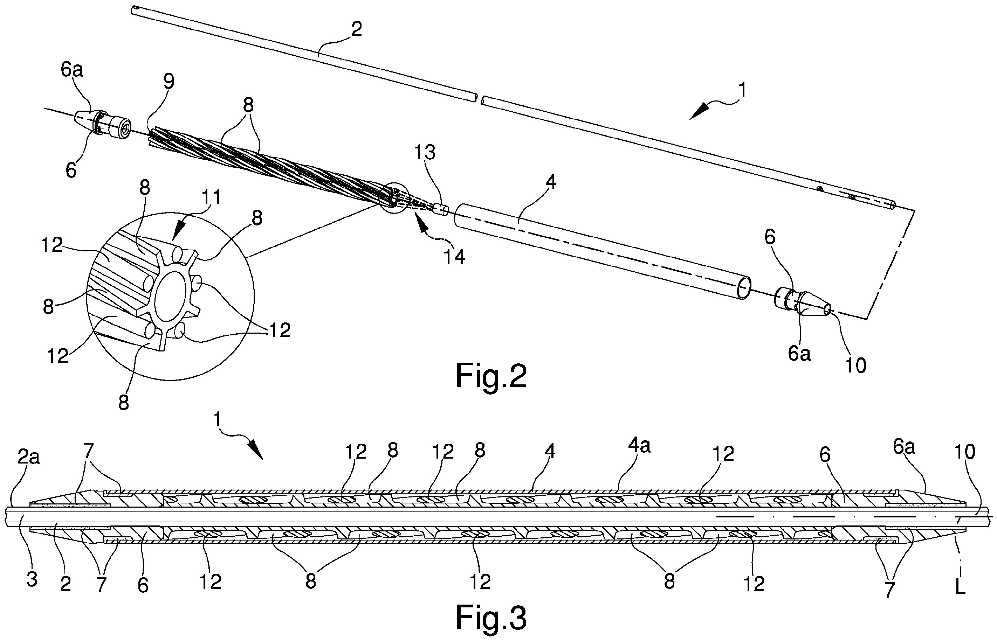

In the embodiment shown in Figures 2 and 3, on the other hand, the ribs 8 are helical and spirally wound around the body 2.

This embodiment has the advantage of stiffening the device 1 in a more balanced manner over all 360° of circumference of the tubular sheath 4.

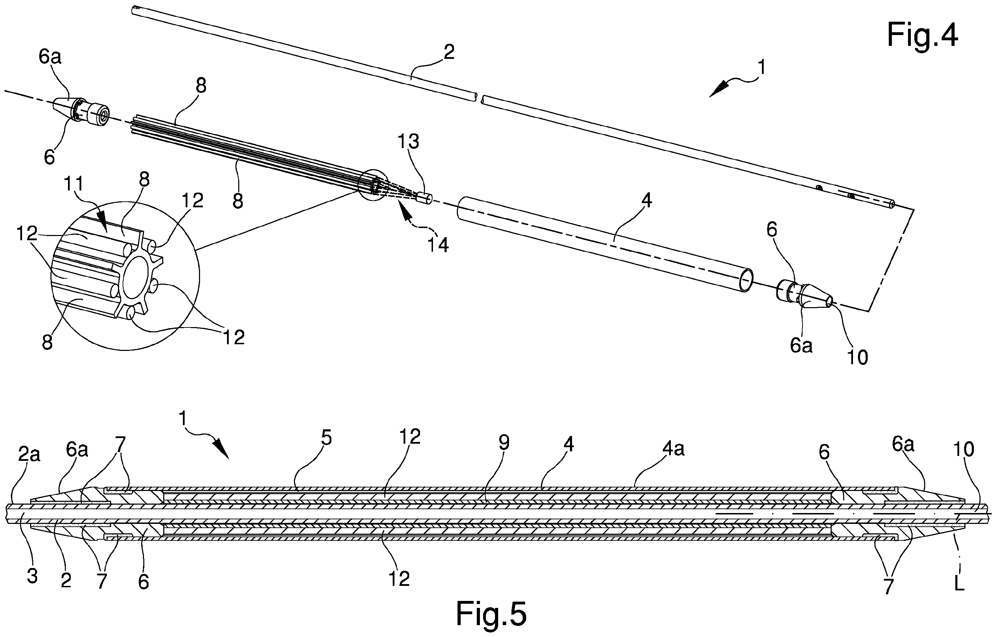

In the particular embodiment shown in Figures 4 and 5, for example, the ribs 8 are rectilinear and parallel to the longitudinal direction L of the body 2.

In the two embodiments shown in the figures, the stiffening element 8, 9 also comprises a tubular section 9 provided with an inner surface fittable around the body 2 and with an outer surface on which the ribs 8 are arranged.

In other words, in the stiffening element 8, 9 shown in the figures, the tubular section 9 is internal and rests on the body 2, while the ribs 8 are external, extending cantilevered from the tubular section 9 and supporting the tubular sheath 4 when it is crushed by the inner walls of the esophagus.

Alternative embodiments cannot however be ruled out wherein the stiffening element 8, 9 comprises a tubular section provided with an outer surface fittable inside the tubular sheath 4 and an inner surface on which the ribs 8 are arranged.

In this case, the tubular section 9 is external and supports the tubular sheath 4, while the ribs 8 are internal and rest on the body 2.

Moreover, other embodiment solutions are possible in which the stiffening element 8, 9 and the tubular sheath 4 are made in a single monolithic body which comprises a tubular section provided with an outer surface adapted to define, in all respects, the tubular sheath 4 and an inner surface on which the ribs 8 are arranged.

In addition, the ribs 8 associated with the tubular section 9 define a substantially U-shaped conformation.

Usefully, the stiffening element 8, 9 is made of an elastomeric material of predetermined hardness, preferably thermoplastic polyurethane (TPU) of which there is a wide range of products available on the market which in their physical and mechanical properties such as hardness, modulus of elasticity, strength, etc.

In actual facts, it is possible to make available to the doctor treating the patient a set of devices 1 having differentiated rigidity, among which is chosen the one that, not only for its size but also for its rigidity, is best suited for use and offers the best resistance to the passage of food through the esophagus according to the patient's actual condition and the progress of the rehabilitation therapy.

According to the invention, the device 1 comprises at least one waveguide 11 housed inside the interspace 5 and configured to drive at least one light radiation R inside it, wherein the light radiation diffuses into the esophagus. Preferably, the waveguide 11 is associated with the stiffening element 8, 9.

The aforementioned light radiation R has a wavelength in the range of 450-475 nm.

This particular expedient is of paramount importance and allows inducing photobiostimulation and, therefore, stimulating the endogenous mechanisms of cell repair.

Additionally, the device 1 comprises at least one light source 15 of the light radiation R.

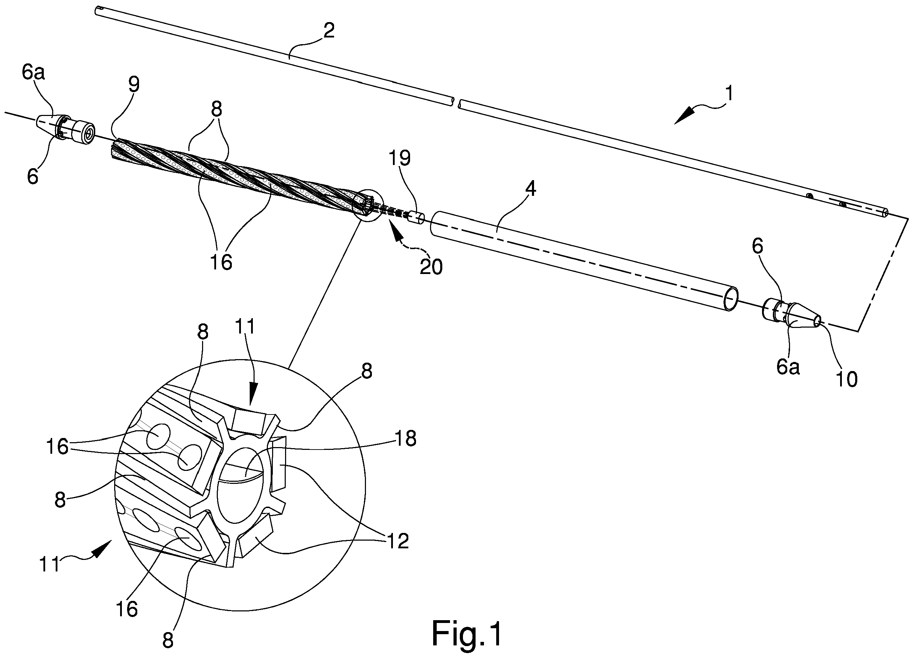

According to the invention, as shown in Figure 1, the light source 15 comprises a plurality of emitting devices 16 coupled to the waveguide 11.

Preferably, the emitting devices 16 are of the LED type.

It cannot however be ruled out from the scope of the present disclosure that the emitting devices are of the type of any electronic device for the emission of a light radiation such as, e.g., a laser or the like.

According to the invention, the emitting devices 16 are arranged adjacent to and aligned with each other along the longitudinal direction L of extension of the body 2 and positioned between the ribs 8.

According to a second and a third embodiment shown in Figures 2-5, the device 1 comprises just one light source 15 placed at input at one end of the waveguide 11, which, in this way, diffuses light along its entire length.

In this case, the light source 15 may be of the LED, laser type or the like.

Additionally, the light source 15 is provided with on/off means adapted to enable it to be switched on and/or off as needed by operational requirements.

According to the first embodiment, the light source 15 comprises power supply means 17 housed inside a housing interspace 18 made internally to the tubular section 9.

The power supply means 17 are operatively connected to each emitting device 16.

In this case, the power supply means 17 define a beam 19 which comes out of the tubular section 9 and is connected to a connection element 20.

For example, the linking element 13 comprises a connector configured to allow the beam 19 to pass through the tubular sheath 4.

Preferably, the connector is a Y-shaped connector.

In this way, the beam 19 comes out of the device 1 through the opening 10; this means that the beam 19 is completely housed inside the housing interspace 18 and the tubular sheath 4, thus avoiding increasing the relevant volumetric size and, consequently, causing discomfort and inconvenience to the patient.

At the same time, according to the second embodiment, the light source 15 comprises charging means of the type of a battery and signaling means for signaling the state of charge of the latter.

In this case, the light source 15 is associated with the waveguide 11 externally to the tubular sheath 4.

With reference to the expression "light source" 15, reference is made to any type of source which is adapted to diffuse inside the waveguide **11** the light radiation R so as to allow it to be conveyed inside the waveguide itself.

According to the second embodiment of the device 1, the waveguide **11** consists of an optical fiber.

Preferably, the device 1 comprises a plurality of waveguides **11** associated with the ribs 8.

According to a second and a third embodiment shown in Figures 2-5, the device 1 comprises a plurality of optical fibers.

In more detail, each optical fiber is positioned between the ribs 8.

Preferably, the optical fibers 12 are fully housed in the space defined between each pair of ribs 8.

In other words, each optical fiber is positioned between a pair of ribs 8.

Additionally, each optical fiber 12 extends along the entire length of the ribs 8, in this case along the entire length of the tubular sheath 4; this allows for homogeneous diffusion of the light radiation R emitted by the optical fibers 12.

The plurality of optical fibers 12 defines a beam 14 of optical fibers 12 coming out of the device 1.

In the present case, the light source 15 is associated with the fiber beam 14 externally to the device 1.

In detail, the optical fibers 12 are connected together by interposition of a linking element 13.

For example, the linking element 13 comprises a connector configured to allow the beam 14 of optical fibers 12 to pass through the tubular sheath 4. Preferably, the connector is a Y-shaped connector.

In this way, the beam 14 of optical fibers 12 comes out of the device 1 through the opening 10; this means that the beam 14 of optical fibers 12 is completely housed inside the tubular sheath 4, thus avoiding increasing the relevant volumetric size and, consequently, causing discomfort and inconvenience to the patient.

Finally, the device 1 also comprises one or more radiopaque markers, not shown in detail in the figures, adapted to allow the exact position of the device inside the esophagus to be identified from the outside.

Such markers may be located inside the walls of the body 2, of the connection elements 6 and of the stiffening element 8, 9 or, alternatively, they may be housed in a portion of the interspace 5 or embedded in the gaps between the connection elements 6 and the body 2 or between the connection elements 6 and the stiffening element 8, 9.

The operation of the device is as follows.

The device 1 is applied to the patient by means of a known technique involving the insertion, using the working channel of a previously used endoscope, of a rigid-type guiding wire.

When the endoscope is removed and the stenotic esophagus tract is dilated with the appropriate technique, the device 1 is made to move forward along the guiding wire and its position is checked by means of appropriate radiological instrumentation which allows the display of the radiopaque markers mounted on the device itself.

The end of the device 1 coming out of the oral cavity is then made to pass through the retropharynx and made to exit through a nostril.

The position of the body 2 inside the esophagus may, e.g., be blocked by applying a naso-gastric tube, of a type known in itself, to the patient.

At this point, the beam 14 of optical fibers 12 coming out of the device 1 is associated with the light source 15.

This means that a light source 15 may be used for different devices for the treatment of esophageal stenosis according to the present invention.

It has in practice been ascertained that the described invention achieves the intended objects.

It should be underlined that the particular expedient of providing for the presence of LED emitting devices or optical fibers placed between the ribs of the stiffening element allows diffusing a blue light beam inside the patient's esophagus, allowing the healing process of stenosis to be significantly accelerated, thus decreasing the time the device itself stays inside the esophagus.

In addition, the present device for the treatment of esophageal stenosis, thanks to the presence of the stiffening element provided with ribs, allows obtaining an effective action throughout the period in which it is applied to the patient, while reducing the pain to the patients themselves.

Additionally, the present invention allows food to pass between the device and the inner walls of the esophagus while causing no discomfort to the patient, thus facilitating full recovery without irritating or injuring the esophagus.

## Claims

1. Device (1) for the treatment of esophageal stenosis, comprising:
- at least one body (2) of elongated shape intended to be inserted inside the esophagus of a patient;
- at least one tubular sheath (4) mounted around said body (2) to define an interspace (5) between said body (2) and said tubular sheath (4);
- at least one stiffening element (8, 9) housed in said interspace (5) and comprising a plurality of ribs (8) extending along said interspace (5);
**characterized by** the fact that it comprises at least one waveguide (11) housed inside said interspace (5) and configured to drive at least one light radiation (R) inside it, said light radiation diffusing into said esophagus, and by the fact that it comprises at least one light source (15) of said light radiation (R), said light source (15) comprising a plurality of emitting devices (16) coupled to said waveguide (11), said emitting devices (16) being arranged adjacent to and aligned with each other along the longitudinal direction (L) of extension of said body (2) and positioned between said ribs (8).

2. Device (1) according to claim 1, **characterized by** the fact that said waveguide (11) is associated with said stiffening element (8, 9).

3. Device (1) according to one or more of the preceding claims, **characterized by** the fact that said waveguide (11) is associated with said ribs (8).

4. Device (1) according to one or more of the preceding claims, **characterized by** the fact that it comprises a plurality of waveguides (11) associated with said ribs (8).

5. Device (1) according to one or more of the preceding claims, **characterized by** the fact that said light radiation has a wavelength comprised in the range of 450-475 nm.

6. Device (1) according to one or more of the preceding claims, **characterized by** the fact that said ribs (8) are rectilinear and parallel to the longitudinal direction (L) of said body (2).

7. Device (1) according to one or more of claims 1-5, **characterized by** the fact that said ribs (8) are helical and spirally wound around said body (2).

8. Device (1) according to one or more of the preceding claims, **characterized by** the fact that said stiffening element (8, 9) comprises a tubular section (9) provided with an inner surface fittable around said body (2) and with an outer surface on which said ribs (8) are arranged.

9. Device (1) according to one or more of claims 1 to 7, **characterized by** the fact that said stiffening element (8, 9) comprises a tubular section provided with an outer surface fittable inside said tubular sheath (4) and an inner surface on which said ribs (8) are arranged.

10. Device (1) according to one or more of the preceding claims, **characterized by** the fact that said body (2) has a substantially circular cross-section and that said tubular sheath (4) is arranged substantially coaxial to said body (2).

11. Device (1) according to one or more of the preceding claims, **characterized by** the fact that it comprises at least one connection element (6) of said tubular sheath (4) to said body (2).

12. Device (1) according to claim 11, **characterized by** the fact that it comprises at least two of said connection elements (6) associated with the axial ends of said tubular sheath (4) and shaped so as to taper the outer surface of said tubular sheath (4) to the outer surface of said body (2).

## Patentansprüche

1. Vorrichtung (1) zur Behandlung von Ösophagusstenose, umfassend:
- mindestens einen Körper (2) mit länglicher Form, der dazu bestimmt ist, in die Speiseröhre eines Patienten eingeführt zu werden;
- mindestens eine röhrenförmige Hülle (4), die um den Körper (2) herum angebracht ist, um einen Zwischenraum (5) zwischen dem Körper (2) und der röhrenförmigen Hülle (4) zu definieren;
- mindestens ein Versteifungselement (8, 9), das in dem Zwischenraum (5) untergebracht ist und eine Vielzahl von Rippen (8) umfasst, die sich entlang des Zwischenraums (5) erstrecken;
**dadurch gekennzeichnet, dass** sie mindestens einen Wellenleiter (11) umfasst, der in dem Zwischenraum (5) untergebracht und ausgebildet ist, mindestens eine Lichtstrahlung (R) in seinem Inneren zu leiten, wobei die Lichtstrahlung in die Speiseröhre streut, und dadurch, dass sie mindestens eine Lichtquelle (15) der Lichtstrahlung (R) umfasst, wobei die Lichtquelle (15) eine Vielzahl von mit dem Wellenleiter (11) gekoppelten Aussendevorrichtungen (16) umfasst, wobei die Aussendevorrichtungen (16) zueinander benachbart und ausgerichtet entlang der Längsrichtung (L) einer Erstreckung des Körpers (2) angeordnet und zwischen den Rippen (8) positioniert sind.

2. Vorrichtung (1) nach Anspruch 1, **dadurch gekennzeichnet, dass** der Wellenleiter (11) mit dem Versteifungselement (8, 9) verbunden ist.

3. Vorrichtung (1) nach einem oder mehreren der vorhergehenden Ansprüche, **dadurch gekennzeichnet, dass** der Wellenleiter (11) mit den Rippen (8) verbunden ist.

4. Vorrichtung (1) nach einem oder mehreren der vorhergehenden Ansprüche, **dadurch gekennzeichnet, dass** sie eine Vielzahl von Wellenleitern (11) umfasst, die mit den Rippen (8) verbunden sind.

5. Vorrichtung (1) nach einem oder mehreren der vorhergehenden Ansprüche, **dadurch gekennzeichnet, dass** die Lichtstrahlung eine Wellenlänge im Bereich von 450 bis 475 nm aufweist.

6. Vorrichtung (1) nach einem oder mehreren der vorhergehenden Ansprüche, **dadurch gekennzeichnet, dass** die Rippen (8) geradlinig und parallel zu der Längsrichtung (L) des Körpers (2) sind.

7. Vorrichtung (1) nach einem oder mehreren der Ansprüche 1 bis 5, **dadurch gekennzeichnet, dass** die Rippen (8) schraubenförmig und spiralförmig um den Körper (2) gewunden sind.

8. Vorrichtung (1) nach einem oder mehreren der vorhergehenden Ansprüche, **dadurch gekennzeichnet, dass** das Versteifungselement (8, 9) einen rohrförmigen Abschnitt (9) umfasst, der mit einer Innenfläche, die um den Körper (2) herum angebracht werden kann, und mit einer Außenfläche versehen ist, auf der die Rippen (8) angeordnet sind.

9. Vorrichtung (1) nach einem oder mehreren der Ansprüche 1 bis 7, **dadurch gekennzeichnet, dass** das Versteifungselement (8, 9) einen rohrförmigen Abschnitt umfasst, der mit einer Außenfläche, die in die rohrförmige Hülle (4) angebracht werden kann, und einer Innenfläche versehen ist, auf der die Rippen (8) angeordnet sind.

10. Vorrichtung (1) nach einem oder mehreren der vorhergehenden Ansprüche, **dadurch gekennzeichnet, dass** der Körper (2) einen im Wesentlichen kreisförmigen Querschnitt aufweist und dass die rohrförmige Hülle (4) im Wesentlichen koaxial zu dem Körper (2) angeordnet ist.

11. Vorrichtung (1) nach einem oder mehreren der vorhergehenden Ansprüche, **dadurch gekennzeichnet, dass** sie mindestens ein Verbindungselement (6) der röhrenförmigen Hülle (4) mit dem Körper (2) umfasst.

12. Vorrichtung (1) nach Anspruch 11, **dadurch gekennzeichnet, dass** sie mindestens zwei der Verbindungselemente (6) umfasst, die mit den axialen Enden der röhrenförmigen Hülle (4) verbunden sind und so geformt sind, dass sie die Außenfläche der röhrenförmigen Hülle (4) zur Außenfläche des Körpers (2) hin verjüngen.

## Revendications

1. - Dispositif (1) pour le traitement de la sténose œsophagienne, comprenant :
- au moins un corps (2) de forme allongée destiné à être introduit à l'intérieur de l'œsophage d'un patient ;
- au moins une gaine tubulaire (4) montée autour dudit corps (2) pour définir un espace intermédiaire (5) entre ledit corps (2) et ladite gaine tubulaire (4) ;
- au moins un élément raidisseur (8, 9) logé dans ledit espace intermédiaire (5) et comprenant une pluralité de nervures (8) s'étendant le long dudit espace intermédiaire (5) ;
**caractérisé par le fait qu'**il comprend au moins un guide d'ondes (11) logé à l'intérieur dudit espace intermédiaire (5) et configuré pour conduire au moins un rayonnement lumineux (R) à l'intérieur de celui-ci, ledit rayonnement lumineux se diffusant dans ledit œsophage, et **par le fait qu'**il comprend au moins une source de lumière (15) dudit rayonnement lumineux (R), ledite source de lumière (15) comprenant une pluralité de dispositifs émetteurs (16) couplés audit guide d'ondes (11), lesdits dispositifs émetteurs (16) étant disposés adjacents et alignés entre eux le long de la direction longitudinale (L) d'étendue dudit corps (2) et positionnés entre lesdites nervures (8).

2. - Dispositif (1) selon la revendication 1, **caractérisé par le fait que** ledit guide d'ondes (11) est associé audit élément raidisseur (8, 9).

3. - Dispositif (1) selon une ou plusieurs des revendications précédentes, **caractérisé par le fait que** ledit guide d'ondes (11) est associé auxdites nervures (8).

4. - Dispositif (1) selon une ou plusieurs des revendications précédentes, **caractérisé par le fait qu'**il comprend une pluralité de guides d'ondes (11) associés auxdites nervures (8).

5. - Dispositif (1) selon une ou plusieurs des revendications précédentes, **caractérisé par le fait que** ledit rayonnement lumineux a une longueur d'onde comprise dans la plage de 450 à 475 nm.

6. - Dispositif (1) selon une ou plusieurs des revendications précédentes, **caractérisé par le fait que** lesdites nervures (8) sont rectilignes et parallèles à la direction longitudinale (L) dudit corps (2).

7. - Dispositif (1) selon une ou plusieurs des revendications 1 à 5, **caractérisé par le fait que** lesdites nervures (8) sont hélicoïdales et enroulées en spirale autour dudit corps (2).

8. - Dispositif (1) selon une ou plusieurs des revendications précédentes, **caractérisé par le fait que** ledit élément raidisseur (8, 9) comprend une section tubulaire (9) comportant une surface interne apte à s'adapter autour dudit corps (2) et une surface externe sur laquelle sont disposées lesdites nervures (8).

9. - Dispositif (1) selon une ou plusieurs des revendications 1 à 7, **caractérisé par le fait que** ledit élément raidisseur (8, 9) comprend une section tubulaire comportant une surface externe apte à s'ajuster à l'intérieur de ladite gaine tubulaire (4) et une surface interne sur laquelle sont disposées lesdites nervures (8).

10. - Dispositif (1) selon une ou plusieurs des revendications précédentes, **caractérisé par le fait que** ledit corps (2) présente une section transversale sensiblement circulaire et que ladite gaine tubulaire (4) est disposée sensiblement coaxialement audit corps (2).

11. - Dispositif (1) selon une ou plusieurs des revendications précédentes, **caractérisé par le fait qu'**il comprend au moins un élément de liaison (6) de ladite gaine tubulaire (4) audit corps (2).

12. - Dispositif (1) selon la revendication 11, **caractérisé par le fait qu'**il comprend au moins deux desdits éléments de liaison (6) associés aux extrémités axiales de ladite gaine tubulaire (4) et conformés de manière à effiler la surface externe de ladite gaine tubulaire (4) vers la surface externe dudit corps (2).
